# EUROPÄISCHE PATENTSCHRIFT

(11) **EP 1 143 950 B1**
(45) Veröffentlichungstag und Bekanntmachung des Hinweises auf die Patenterteilung: **09.03.2005**
(21) Anmeldenummer: 99939884.5
(22) Anmeldetag: 03.09.1999
(51) Int. Cl.: A61K 47/14, A61P 31/10

(54) **TOPISCH ANWENDBARE MITTEL GEGEN NAGELPILZERKRANKUNGEN**
TOPICAL APPLICATION PRODUCTS AGAINST ONYCHOMYCOSES
PRODUITS POUR L'APPLICATION LOCALE CONTRE LES ONYCHOMYCOSES

(30) Priorität: 10.09.1998 EP 98117114; 05.03.1999 EP 99104466
(43) Veröffentlichungstag der Anmeldung: 17.10.2001
(73) Patentinhaber: BioEqual AG, 4132 Muttenz (CH)
(72) Erfinder: MEYER, Hans, CH-4125 Riehen (CH); WASMER, Hermann, CH-4125 Riehen (CH)
(86) Internationale Anmeldenummer: PCT/CH1999/000409
(87) Internationale Veröffentlichungsnummer: WO 2000/015202

(56) Entgegenhaltungen:
- EP-A- 0 745 372
- EP-A- 0 770 399
- WO-A-96/40047
- US-A- 4 710 497
- DATABASE EPODOC [Online] European Patent Office; XP002124817 & CN 1 183 961 A (NO 1 HOSPITAL ATTACHED TO SUZH) 10. Juni 1998 (1998-06-10)
- PATENT ABSTRACTS OF JAPAN vol. 11, no. 96 (C-412), 26. März 1987 (1987-03-26) & JP 61 246113 A (SHISEIDO CO LTD), 1. November 1986 (1986-11-01)

## Beschreibung

Die vorliegende Erfindung betrifft wasserfreie, topisch anwendbare Mittel zur Behandlung von Nagelerkrankungen und zur Nagelpflege, die eine oder mehrere Wirksubstanzen, C₁-C₄-Alkylester der Milchsäure, der Äpfelsäure, der Weinsäure oder der Zitronensäure als Carrier und gegebenenfalls physiologisch verträgliche Hilfsstoffe enthalten. Die Erfindung betrifft ebenfalls ein Verfahren zur Herstellung dieser Mittel und ihre Verwendung.

Die direkte topische Behandlung von Nagelerkrankungen und die Nagelpfiege verlaufen praktisch nebenwirkungsfrei, sind sehr einfach durchzuführen und verursachen nur geringe Kosten. Das wesentliche Problem der direkten topischen Anwendung von Nagelzubereitungen besteht jedoch darin, die Wirkstoffe, einschliesslich Nähr- und Aufbaustoffe, in ausreichender Menge durch den Nagel hindurch in die tiefer gelegenen Gewebeschichten und in die Nagelwurzel zu bringen und die dort vorhandenen Erreger vollständig abzutöten respektive den Nagel mit Nähr- und Aufbaustoffen zu versorgen. Mit herkömmlichen Mitteln gelingt es zwar, die Symptome durch direkte topische Behandlung zum Abklingen zu bringen, sie treten jedoch nach Abbruch der Behandlung in den meisten Fällen wieder auf.

Es wurde bereits vorgeschlagen, den Behandlungserfolg bei der direkten topischen Anwendung von Wirkstoffen dadurch zu verbessern, dass man die Wirkstoffe zusammen mit einem sogenannten Carrier, d. h. einer Substanz, die neben einem guten Lösungsvermögen für den Wirkstoff auch ein gutes Penetrationsvermögen durch die Nagelsubstanz und die Fähigkeit besitzt, die Wirksubstanz durch das Nagelgewebe zu transportieren, zur Anwendung brachte. So werden beispielsweise in der EP-A-0 503 988 Arzneimittel zur Behandlung von Onychomykosen beschrieben, die neben einer antimykotischen Wirksubstanz und einem wässerigen Medium, in dem das Antimykotikum wenigstens teilweise löslich ist, mindestens eine hydrophile, die Penetration des Antimykotikums durch den Nagel fördernde Substanz enthalten. Als penetrationsfördernde Substanz wird neben einer grossen Zahl von Verbindungen auch Milchsäureethylester genannt. Das in EP-A-0 503 988 beschriebene Formulierungsprinzip ist wegen der für die Wirksubstanz postulierten partiellen Wasserlöslichkeit nur für eine beschränkte Zahl von Wirksubstanzen anwendbar. Ausserdem können nach diesem Prinzip wegen des Wassergehaltes der Formulierungen bei Verwendung von hydrolysierbaren Verbindungen als penetrationsfördernde Substanzen, wie Milchsäureethylester, keine stabilen Formulierungen hergestellt werden, da sich solche Verbindungen während der Lagerzeit durch Hydrolyse zersetzen.

Es gibt bisher kein zufriedenstellendes Mittel zur topischen Behandlung von Nagelerkrankungen oder zur topischen Nagelpflege, das einen Carrier enthält, der den Transport einer für einen dauerhaften Behandlungserfolg erforderlichen Menge an Wirksubstanz durch den Nagel in das darunter liegende Nagelbett und zur Nagelwurzel (Matrix) gewährleistet.

Es ist daher die Aufgabe der vorliegenden Erfindung, die im Zusammenhang mit der topischen Behandlung von Nagelerkrankungen und der topischen Nagelpflege bestehenden Probleme zu lösen und pharmazeutische respektive kosmetische Mittel bereitzustellen, welche einen raschen und dauerhaften Behandlungserfolg ermöglichen.

Gemäss vorliegender Erfindung werden wasserfreie, topisch anwendbare Mittel zur Behandlung von Nagelerkrankungen und zur Nagelpflege vorgeschlagen, die sich wie folgt zusammensetzen:
a) eine oder mehrere Wirksubstanzen
b) einen oder mehrere C₁-C₄-Alkylester der Milchsäure, der Äpfelsäure, der Weinsäure oder der Zitronensäure als Carrier und
c) gegebenenfalls physiologisch verträgliche Hilfsstoffe.
Für die erfindungsgemässen wasserfreien, topisch anwendbaren Mittel kommen grundsätzlich alle Wirksubstanzen synthetischer und natürlicher Herkunft in Betracht, die bei Nagel- und Periungual-Erkrankungen wirksam sind. Ferner kommen als Wirksubstanzen Nähr- und Aufbaustoffe in Betracht, die bei der Nagelpflege effektiv sind.

Geeignete Wirksubstanzen, die in den erfindungsgemässen Mitteln enthalten sein können, sind Antimykotika synthetischer und natürlicher Herkunft, Antibiotika, Antiseptika, Kortikosteroide, Nähr- und Aufbaustoffe sowie Kombinationen der genannten Wirksubstanzen. Spezielle Beispiele für solche Wirksubstanzen sind:
- Antimykotika und ihre physiologisch verträglichen Salze, wie z.B. (±)-cis-2,6-Dimethyl-4-[2-methyl-3-(*p*-*tert*-pentyl-phenyl)propyl]morpholin (Amorolfin), Amphotericin, 6-Cyclohexyl-1-hydroxy-4-methyl-2(1H)-pyridinon (Ciclopirox), Bis-phenyl-(2-chlorphenyl)-1-imidazolylmethan (Clotrimazol), 1-[2-(2,4-Dichlorphenyl)-2-(4-chlorbenzyloxy)-ethyl]-imidazol (Econazol), 2,4-Difluor-α,α-bis(1*H*-1,2,4-triazol-1-ylmethyl)benzylalkohol (Fluconazol), 5-Fluorcytosin (Flucytosin), 7-Chlor-trimethoxy-methylspiro-[benzofuran-cyclohexen]-dion (Griseofulvin), 1-[2,4-Dichlor-β-(2,6-dichlorbenzyloxy)-phenethyl]-imidazol (Isoconazol), (±)-1-sec-Butyl-4-{4-[4-(4-{[(2R*,4S*)-2-(2,4-dichlorphenyl)-2-(1,2,4-triazol-1-ylmethyl)-1,3-dioxolan-4-yl]methoxy}phenyl)-1-piperazinyl]phenyl}-4,5-dihydro-1,2,4-triazol-5-on (Itraconazol), (±)-cis-1-Acetyl-4-{4-([2-(2,4-dichlorphenyl)-2-(1H-imidazol-1-ylmethyl)-1,3-dioxolan-4-yl]methoxy)phenyl}piperazin (Ketoconazol), 1-[2,4-Dichlor-β-(2,4-dichlorbenzyloxyl)-phenethyl]-imidazol (Miconazol), (E)-N-Cinnamyl-*N*-methyl-1-naphthylmethylamin (Naftifin), Nystatin, (*E*)-*N*-(6,6-Dimethyl-2-hepten-4-inyl)-*N*-methyl-1-naphthylmethylamin (Terbinafin), 1[2-{(2-Chlor-3-thienyl)methoxy}-2-(2,4-dichlorphenyl)ethyl}-1H-imidazol (Tioconazol), O-2-Naphthyl-N-methyl-N-(3-tolyl)-thiocarbamat (Tolnaftat), α-(2,4-Difluorophenyl)-5-fluoro-β-methyl-α-(1H-1,2,4-triazol-1-ylmethyl)-4-pyrimidinethanol (Voriconazol).
- Antimykotika natürlicher Herkunft, wie beispielsweise ätherische Öle und Pflanzenextrakte.
- Antibiotika und ihre physiologisch verträglichen Salze, wie z.B. α-Amino-4-hydroxybenzylpenicillin (Amoxicillin), D-(-)-α-Aminobenzylpenicillin (Ampicillin), 3,3-Dimethyl-7-oxo-6-phenylacetamido-4-thia-1-azabicyclo-[3.2.0]-heptan-2-carbonsäure (Benzylpenicillin), Benzylpenicillin-Benzathin, 3-Chloro-7-D(2-phenylglycinamido)-cephalosporansäure (Cefaclor), 7β-[D-2-Amino-(4-hydroxy-phenyl)-acetylamino]-3-methyl-cephalosporansäure (Cefadroxil), Aminophenyl-acetamido-methyl-cephalosporansäure (Cefalexin), D(-)-threo-2-dichloracet-amido-1-(4-nitrophenyl)-1,3-propandiol (Chloramphenicol), 1-Cyclopropyl-6-fluor-1,4-dihydro-4-oxo-7-(1-piperazinyl)-3-chinolincarbonsäure (Ciprofloxacin), (Z)-(2R,5R)-3-(2-Hydroxyethyliden)-7-oxo-4-oxa-1-azabicyclo[3.2.0]heptan-2-carbonsäure (Clavulansäure), 7-Chlor-7-desoxy-lincomycin (Clindamycin), 6-Desoxy-5-hydroxytetracyclin (Doxycyclin), 1-Ethyl-6-fluor-1,4-dihydro-4-oxo-7-(1-piperazinyl)-1,8-naphthyridin-3-carbonsäure (Enoxacin), Erythromycin, 3-(2-Chlor-6-fluorphenyl)-5-methyl-4-isoxazolyl-penicillin (Flucloxacillin), Kanamycin, Lincomycin, 7-Dimethylamino-6-desoxy-6-desmethyltetracyclin (Minocyclin), 6-(2-ethoxy-1-naphthamido)-penicillin (Nafcillin), 1-Ethyl-1,4-dihydro-7-methyl-4-oxo-1,8-naphthyridin-3-carbonsäure (Nalidixinsäure), Neomycin, 1-Ethyl-6-fluoro-1,4-dihydro-4-oxo-7-(1-piperazinyl)-3-chinolincarboxylsäure (Norfloxacin), (±)-9-Fluor-2,3-dihydro-3-methyl-10-(4-methyl-1-piperazinyl)-7-oxo-7*H*-pyrido[1,2,3-*de*][1,4]benzoxazin-6-carbonsäure (Ofloxacin), 6-(5-Methyl-3-phenyl-4-isoxazolcarboxamido)penicillansäure (Oxacillin), 6-Phenoxyacetylamino-penicillansäure (Phenoxymethylpenicillin), 4-Dimethylamino-octahydro-pentahydroxy-1,11-dioxo-6-methyl-naphtacen-2-carbamid (Tetracyclin).
- Antiseptika, wie z.B. Alkylbenzyldimethylammoniumchlorid (Benzalkoniumchlorid), N-Benzyl-N,N-dimethyl-2-{2-[p-(1,1,3,3,-tetramethylbuthyl)-phenoxy]-ethoxy}-ethylammoniumhydroxid (Benzethoniumchlorid), Cetyltrimethylammoniumhydroxid (Cetrimoniumbromid), 1,1'-Hexamethylen-bis-[5-(p-chlorphenyl)-biguanid] (Chlorhexidin), N¹,N¹-Dekamethylen-bis-(4-aminochinaldiniumhydroxid) (Dequaliniumchlorid), N-(4-Chlorphenyl)-N'-(3,4-dichlorphenyl)harnstoff (Triclocarban), 5-Chlor-2-(2,4-dichlorphenoxy)phenol (Triclosan).
- Kortikosteroide und ihre physiologisch verträglichen Salze, wie z.B. 9α-Chlor-16β-methylprednisolon (Beclomethason), 9-Fluor-11β, 17,21-trihydroxy-16β-methyl-1,4-pregnadien-3,20-dion (Betamethason), 21-Chlor-9-fluor-11β,17-dihydroxy-16β-methyl-1,4-pregnadien-3,20-dion (Clobetasol), 17,21-Dihydroxy-pregn-4-en-3,11,20-trion (Cortison), 11β,16α,17α,21-Tetrahydroxy-1,4-pregnadien-3,20-dion-16,17-acetonacetal (Desonid), 9-Fluor-11β-17,21-trihydroxy-16α-methylpregna-1,4-dien-3,20-dion (Dexamethason), 9α,11β-Dichloro-6α-fluoro-21-hydroxy-16α,17α-(isopropylidendioxy)-pregna-1,4-dien-3,20-dion (Flucloronid), 6α,9α-Difluor-16α, 17α-isopropylidendioxycorticosteron (Fluocinolonacetonid), 6α,9α-Difluor-16α,17α-isopropylidendioxy-corticosteron-acetat (Fluocinonid), 6α-Fluor-11β,21-dihydroxy-16α,17-isopropylidendioxy-4-pregnen-3,20-dion (Fludroxycortid), 3-(2-chloroethoxy)-9α-fluoro-6-formyl-11β,21-dihydroxy-16α,17α-isopropylidendioxypregna-3,5-dien-20-on (Formocortal), 21-Chlor-9α-fluor-11β-hydroxy-16α, 17α-isopropylidendioxy-4-pregnen-3,20-dion (Halcinonid), 17α-Hydroxycorticosteron (Hydrocortison), 11β,17,21-Trihydroxy-6α-methyl-1,4-pregnadien-3,20-dion (Methylprednisolon), 11β,17,21-Trihydroxy-pregna-1,4-dien-3,20-dion (Prednisolon), 17α,21-Dihydroxy-pregna-1,4-dien-3,11,20-trion (Prednison), 9-Fluor-16α-hydroxyprednisolon (Triamcinolon), Triamcinolon-16α,17α-acetonid (Triamcinolonacetonid).
- Nähr- und Aufbaustoffe, wie z.B. 2-Pyrrolidincarbonsäure (L-Prolin).

Gemäss vorliegender Erfindung bevorzugte Antimykotika sind Bis-phenyl-(2-chlorphenyl)-1-imidazolylmethan (Clotrimazol), 1-[2,4-Dichlor-β-(2,6-dichlorbenzyloxy)-phenethyl]-imidazol (Isoconazol), 2,4-Difluor-α,α-bis(1*H*-1,2,4-triazol-1-ylmethyl)benzylalkohol (Fluconazol), (±)-1-sec-Butyl-4-{4-[4-(4-{[(2R*,4S*)-2-(2,4-dichlorphenyl)-2-(1,2,4-triazol-1-ylmethyl)-1,3-dioxolan-4-yl]methoxy}phenyl)-1-piperazinyl]phenyl}-4,5-dihydro-1,2,4-triazol-5-on (Itraconazol), (±)-cis-1-Acetyl-4-{4-([2-(2,4-dichlorphenyl)-2-(1H-imidazol-1-ylmethyl)-1,3-dioxolan-4-yl]methoxy)phenyl}piperazin (Ketoconazol), 1-[2,4-Dichlor-β-(2,4-dichlorbenzyloxyl)-phenethyl]-imidazol (Miconazol), (*E*)-*N*-(6,6-Dimethyl-2-hepten-4-inyl)-*N*-methyl-1-naphthylmethylamin (Terbinafin), α-(2,4-Difluorophenyl)-5-fluoro-β-methyl-α-(1H-1,2,4-triazol-1-ylmethyl)-4-pyrimidinethanol (Voriconazol).

Gemäss vorliegender Erfindung besonders bevorzugte Antimykotika sind Bis-phenyl-(2-chlorphenyl)-1-imidazolylmethan (Clotrimazol), 1-[2,4-Dichlor-β-(2,6-dichlorbenzyloxy)-phenethyl]-imidazol (Isoconazol), (±)-1-*sec*-Butyl-4-{4-[4-(4-{[(2R*,4S*)-2-(2,4-dichlorphenyl)-2-(1,2,4-triazol-1-ylmethyl)-1,3-dioxolan-4-yl]methoxy}phenyl)-1-piperazinyl]phenyl}-4,5-dihydro-1,2,4-triazol-5-on (Itraconazol), (±)-cis-1-Acetyl-4-{4-([2-(2,4-dichlorphenyl)-2-(1H-imidazol-1-ylmethyl)-1,3-dioxolan-4-yl]methoxy)phenyl}piperazin (Ketoconazol).

Bevorzugte Antimykotika natürlicher Herkunft sind Teebaumöl (Melaleuca alternifolia), Lavendelöl (Lavandula officinalis chaix) und Blattextrakt des Niembaumes (Azadirachta indica). Diese natürlichen Antimykotika können als einzelne Wirkstoffe oder als Kombination mehrerer solcher Wirkstoffe verwendet werden. Eine bevorzugte Wirkstoffkombination ist eine Mischung von Lavendelöl, Teebaumöl und Niembaumblatt-Extrakt.

Bevorzugte Antiseptika , wie z.B. 1,1'-Hexamethylen-bis-[5-(p-chlorphenyl)-biguanid] (Chlorhexidin).

Bevorzugte Kortikosteroide sind 11β, 16α, 17α,21-Tetrahydroxy-1,4-pregnadien-3,20-dion-16,17-acetonacetal (Desonid), 9α,11β-Dichloro-6α-fluoro-21-hydroxy-16α,17α-(isopropylidendioxy)-pregna-1,4-dien-3,20-dion (Flucloronid), 6α,9α-Difluor-16α,17α-isopropylidendioxy-corticosteron (Fluocinolonacetonid), 6α,9α-Difluor-16α,17α-isopropylidendioxy-corticosteron-acetat (Fluocinonid), 6α-Fluor-11β,21-dihydroxy-16α,17-isopropylidendioxy-4-pregnen-3,20-dion (Fludroxycortid), 3-(2-chloroethoxy)-9α-fluoro-6-formyl-11β,21-dihydroxy-16α,17α-isopropylidendioxypregna-3,5-dien-20-on (Formocortal), 21-Chlor-9α-fluor-11β-hydroxy-16α,17α-isopropylidendioxy-4-pregnen-3,20-dion (Halcinonid), Triamcinolon-16α,17α-acetonid (Triamcinolonacetonid).

Spezielle Beispiele für Kombinationen von Wirksubstanzen sind:
- Kombinationen von Kortikosteroiden mit Antimykotika, Antibiotika oder Antiseptika. Eine bevorzugte Kombination ist z. B. (±)-cis-1-Acetyl-4-{4-([2-(2,4-dichlorphenyl)-2-(1H-imidazol-1-ylmethyl)-1,3-dioxolan-4-yl]methoxy)phenyl}-piperazin (Ketoconazol) und 11β,16α,17α, 21-Tetrahydroxy-1,4-pregnadien-3,20-dion-16,17-acetonacetal (Desonid).
- Kombinationen von Antimykotika synthetischer Herkunft mit Antimykotika natürlicher Herkunft. Eine bevorzugte Kombination ist bis-Phenyl-(2-chlorphenyl)-1-imidazolylmethan (Clotrimazol) mit Teebaumöl.
- Kombinationen von verschiedenen Antimykotika natürlicher Herkunft. Eine bevorzugte Kombination ist Lavendelöl, Teebaumöl und Niembaumblatt-Extrakt.
- Kombinationen von 2-Pyrrolidincarbonsäure (L-Prolin) mit einem oder mehreren weiteren Nähr- und Aufbaustoffen, die aus den Gruppen der Aminosäuren, der Vitamine und der Mineralstoffen ausgewählt sind. Bevorzugte Kombinationen von 2-Pyrrolidincarbonsäure (L-Prolin) mit einem oder mehreren Nähr- und Aufbaustoffen sind Kombinationen mit (S)-2,6-Diaminohexansäure (Lysin), (R)-2-Amino-3-mercaptopropionsäure (Cystein), Gelatine, Cis-2-(4-Carboxybutyl)-3,4-ureidotetrahydrothiophen (Biotin), (±)-2,4-Dihydroxy-N-(3-hydroxypropyl)-3,3-dimethylbutyramid (Panthenol), D(+)-2,4-Dihydroxy-N-(3-hydroxypropyl)-3,3-dimethylbutyramid (Dexpanthenol) und anorganische oder organische Calcium-, Magnesium- oder Zinkverbindungen.

Mit L-Prolin wurde ein Aufbaustoff gefunden, der sich zum Nagelaufbau und zur Nagelpflege als besonders geeignet erwiesen hat. L-Prolin wurde bisher lediglich als fakultativer Bestandteil von kosmetischen Mitteln zur Nagelpflege erwähnt, die als wirksame Komponente eine sulfurierte Aminosäure oder ein Derivat der sulfurierten Aminosäure enthalten (EP-A-0 534 810).

Die oben genannten Substanzen werden nachfolgend mit dem entsprechenden, in obiger Aufzählung in Klammern gesetzten Trivialnamen bezeichnet.

Die als Carrier zu verwendenden C₁-C₄-Alkylester umfassen die Methyl-, Ethyl-, n-Propyl, Isopropyl, n-Butyl-, sec.-Butyl, Isobutyl- und tert.-Butylester. Bei den Estern der mehrbasischen Säuren Äpfelsäure, Zitronensäure und Weinsäure können die in den Estergruppen enthaltenen C₁-C₄-Alkylgruppen gleich oder verschieden sein. In den vorgenannten mehrbasischen Säuren können alle Carboxylgruppen oder nur ein Teil der Carboxylgruppen verestert sein. Es kommen daher neben Äpfelsäure- und Weinsäuredi-C₁-C₄-alkylestern auch die entsprechenden Äpfelsäure- und Weinsäuremonoalkylester in Betracht. Von den C₁-C₄-Alkylestern der Zitronensäure sind die entsprechenden Mono-, Diund Trialkylester geeignet. Bevorzugte Ester sind die Ethylester. Weitere bevorzugte Ester sind die Isopropylester. Eine bevorzugte Einzelverbindung ist Milchsäureethylester. Weitere bevorzugte Einzelverbindung sind Äpfelsäurediethylester und Äpfelsäurediisopropylester.

Die erfindungsgemässen topisch anwendbaren Mittel können neben einer oder mehrerer Wirksubstanzen und einem oder mehreren C₁-C₄-Alkylestern der Milchsäure, der Äpfelsäure, der Weinsäure oder der Zitronensäure als Carrier übliche physiologisch verträgliche Hilfsstoffe enthalten. Geeignete Hilfsstoffe dieser Art sind beispielsweise Terpene oder Terpene enthaltende Öle, Alkohole, Ketone, Fettsäureester, Polyglykole, Tenside, Harnstoff, Antioxidantien und Komplexierungsmittel.

Geeignete Terpene sind acyclische, monocyclische und bicyclische Terpene sowie Öle, die diese Terpene enthalten. Beispiele für acyclische Terpene sind acyclische Terpenkohlenwasserstoffe, wie z.B. Myrcen, acyclische Terpenalkohole, wie z.B. Citronellol und Geraniol, sowie acyclische Terpenaldehyde und -ketone, wie z.B. Citral, α-Jonon und β-Jonon. Beispiele für monocyclische Terpene sind monocyclische Terpenkohlenwasserstoffe, wie z.B. α-Terpinen, γ-Terpinen und Limonen, monocyclische Terpenalkohole, wie z.B. Thymol, Menthol, Cineol und Carvacrol, sowie monocyclische Terpenketone, wie z.B. Menthon und Carvon. Beispiele für bicyclische Terpene sind Terpene aus der Carangruppe, wie z.B. Caron, Terpene aus der Pinangruppe, wie z.B. α-Pinen und β-Pinen, sowie Terpene aus der Bornangruppe, wie z.B.Campher und Borneol. Besonders geeignete Terpene sind monocyclische Terpenalkohole, wie z.B. Thymol und Menthol. Beispiele für geeignete Öle, dieTerpene enthalten, sind Pfefferminzöl, Cardamomenöl, Geraniumöl, Rosenöl, Thujaöl und Thymianöl. Besonders geeignete Öle sind Pfefferminzöl, Lavendelöl und Thymianöl.

Geeignete Alkohole sind verzweigte oder unverzweigte Alkohole mit 1 bis 3 Hydroxygruppen und 2 bis 6 Kohlenstoffatomen, wobei die Hydroxygruppen teilweise oder vollständig verethert und verestert sein können. Speziell geeignete Alkohole sind Ethanol, 1-Propanol, 2-Propanol (Isopropanol), 1,2-Propandiol (Propylenglykol), 2-Phenylethanol (Phenylethylalkohol), 1-Butanol (Butylalkohol), Ethylenglycolmonomethylether (Methoxyethanol), Ethylenglycolmonophenylether (Phenoxyethanol), 1,2,3-Trihydroxypropan (Glycerin), Ethylacetat, Butylacetat, Glycerindiacetat (Diacetin) und Glycerintriacetat (Triacetin).

Als geeignete Ketone kommen beispielsweise Aceton und Methylethylketon (2-Butanon) in Betracht.

Als Fettsäureester sind Ester von gesättigten und ungesättigten, verzweigten und unverzweigten Fettsäuren mit 8 bis 21 Kohlenstoffatomen geeignet, wobei die Alkoholkomponente verzweigte und unverzweigte Alkohole mit 1 bis 6 Kohlenstoffatomen umfasst. Speziell geeignete Fettsäureester sind Tridecancarbonsäureisopropylester, Tetradecancarbonsäureisopropylester (isopropylmyristat), Pentadecancarbonsäuremethylester und 9-Octadecensäureglycerinmonoester (Glycerinmonooleat).

Ein geeignetes Polyglykol ist beispielsweise Polyglykol 400.

Geeignete Tenside sind beispielsweise nicht ionogene oberflächenaktive Substanzen. Speziell geeignete Tenside sind Partialfettsäureester des Sorbitans (Span), Partialfettsäureester des Polyoxyethylensorbitans (Tween), Fettsäureester des Polyoxyethylens (Myrj) und Fettalkoholether des Polyoxyethylens (Brij).

Geeignete Antioxidantien sind beispielsweise Butylhydroxytoluol (BHT), Butyl-4-methoxyphenol (BHA), Tocopherole und Ascorbate.

Als Komplexierungsmittel eignen sich beispielsweise Ethylendiamintetraessigsäure (EDTA) und Dinatrium-Ethylendiamintetraessigsäure (Na₂-ETDA).

Als erfindungsgemässe topisch anwendbare Mittel kommen zum Beispiel Lösungen, Tinkturen, Emulsionen, Gele, Salben, Cremes und Pasten in Betracht. Bevorzugte topische Darreichungsformen sind Lösungen.

Die Erfindung betrifft ferner ein Verfahren zur Herstellung der erfindungsgemässen topisch anwendbaren Mitteln, welches dadurch gekennzeichnet ist, dass man Milchsäureethylester und einen oder mehrere Hilfsstoffe homogen vermischt, anschliessend eine oder mehrere Wirksubstanzen in der Mischung unter Rühren und gegebenenfalls Erwärmen (maximal 80°C) löst und solange weiterrührt, bis eine homogene Wirkstofflösung entstanden ist. Die erhaltene Lösung wird bevorzugt direkt als solche zur topischen Applikation verwendet. Die Lösung kann aber auch unter Zugabe von weiteren physiologisch verträglichen Formulierungshilfsstoffen mittels konventionellen Lösungs-, Misch- und Suspendierverfahren in eine andere topische Darreichungsform gebracht werden.

Bevorzugt werden die erfindungsgemässen topisch anwendbaren Mittel in Form von Lösungen verwendet. Bevorzugte topisch anwendbare Mittel enthalten gemäss vorliegender Erfindung

| | |
|---|---|
| 0,01 bis 20 Gew.-% | einer oder mehrerer Wirksubstanzen, |
| 1 bis 99,99 Gew.-% | eines oder mehrerer C₁-C₄-Alkylester der Milchsäure, der Äpfelsäure, der Weinsäure oder der Zitronensäure und |
| 0 bis 98,99 Gew.-% | eines oder mehrerer physiologisch verträglicher Hilfsstoffe. |

Die Erfindung betrifft weiterhin die Verwendung der erfindungsgemässen topisch anwendbaren Mittel zur Behandlung, Prävention, Nachbehandlung oder unterstützenden Behandlung von Nagelerkrankungen und periungualen Erkrankungen sowie zur Nagelpflege. Ausserdem betrifft die vorliegende Erfindung die Verwendung der erfindungsgemässen Mittel zur Behandlung von Pilzinfektionen von Hufen, Klauen und Krallen von Haus- und Nutztieren.

Topisch anwendbare Mittel enthaltend Antimykotika eignen sich beispielsweise für folgende Indikationen:
- Behandlung, Prävention und Nachbehandlung von Onychomykosis, verursacht durch Dermatophyten, Hefen oder Schimmelpilze oder Mischinfektionen
- Behandlung, Prävention und Nachbehandlung von Nagelpilzinfektionen bei Patienten mit Psoriasis, Diabetes oder AIDS
- Unterstützung der Behandlung von periungualen Nagelinfektionen, wie z.B. Candida paronychium.

Topisch anwendbare Mittel enthaltend Antibiotika eignen sich beispielsweise für folgende Indikationen:
- Unterstützung der Behandlung und/oder Prävention von Nagel- und periungualen Infektionen, verursacht durch Bakterien.

Topisch anwendbare Mittel enthaltend Antiseptika eignen sich beispielsweise für folgende Indikationen:
- Behandlung und Prävention von Nagel- und periungualen Infektionen, verursacht durch unspezifische oder nicht identifizierte Erreger.

Topisch anwendbare Mittel enthaltend Kortikosteroide oder Kombinationen von Kortikosteroiden mit Antimykotika, Antibiotika oder Antiseptika eignen sich beispielsweise für folgende Indikationen:
- Behandlung, Prävention, Nachbehandlung oder unterstützende Behandlung von Nagel-Psoriasis oder anderen entzündlichen Nagel- und periungualen Zuständen.

Die erfindungsgemässen pharmazeutischen, topisch anwendbaren Mittel eignen sich zur Behandlung von Nagelerkrankungen und periungualen Erkrankungen an Zehen- und Fingernägeln, sowie zur Behandlung von Erkrankungen der Hufe, Klauen und Krallen von Haus- und Nutztieren. Die Applikationsfrequenz der pharmazeutischen Mittel richtet sich nach dem Ausmass und der Lokalisation der Erkrankung. Im allgemeinen genügt eine ein- bis dreimalige Anwendung pro Tag. Die Lösung wird hierbei direkt auf den kranken Nagel bezw. den Huf, die Klaue oder die Kralle und bei Bedarf auf die ebenfalls betroffenen umliegenden Hautbezirke aufgetragen. Die Therapie sollte nach Symptomfreiheit noch etwa zwei Wochen weitergeführt werden, um einen Rückfall zu verhindern.

Die erfindungsgemässen kosmetischen, topisch anwendbaren Mittel, enthaltend einen oder mehrere Nähr- und Aufbaustoffe, eignen sich zur Nagelpflege, wie zum Beispiel bei Nagelatrophien an Zehen- und Fingernägeln. Nagelatrophien beinhalten beispielsweise brüchige, spröde und dünne Nägel sowie punktförmige oder streifige weisse Flecken. Die Zubereitung wird auf den oder die kosmetisch unschönen Nägel und bei Bedarf auch auf umliegende Hautbezirke aufgetragen. Die Applikationsfrequenz der Zubereitung richtet sich nach dem Ausmass und der Lokalisation der Atrophie. Im allgemeinen genügt eine ein-zweimalige Anwendung pro Tag.

Die erfindungsgemässen topisch anwendbaren Mittel haben den Vorteil, dass sie den erkrankten Nagel mitsamt den Wirksubstanzen innerhalb von wenigen Tagen durchdringen und im Nagelbett und an der Nagelwurzel ihre Wirkung entfalten können. Durch den rascheren Wirkungseintritt und die bessere Penetration ist die Behandlung von Nagelkrankheiten in der Regel nach etwa zwei bis drei Monaten abgeschlossen. Dadurch wird die Patienten-Compliance deutlich verbessert, da die bei anderen Behandlungsmethoden erforderliche lange Behandlungsdauer wesentlich verkürzt wird. Bei erkrankter Haut, insbesondere bei periungualen Hautbezirken, tritt der Heilungsprozess beziehungsweise der Pflegeeffekt rascher ein, da der Wirkstoff effizient und schnell in die Haut eindringt. Die Nagelpflege sollte in der Regel einen Monat lang durchgeführt werden. Zur Erhaltung der gesunden Nagelsubstanz kann das Nagelpflegeprodukt auch über einen längeren Zeitraum angewendet werden.

Die vorliegende Erfindung wird durch folgende Beispiele veranschaulicht:

### Beispiel 1: Clotrimazol-Lösung 1 %

| | |
|---|---|
| Milchsäureethylester | 20,0 ml |
| Harnstoff | 2,0 g |
| Clotrimazol | 1,0 g |
| Milchsäureethylester | ad 100,0 ml |

Harnstoff wird in 20 ml Milchsäureethylester unter Rühren und Erwärmen (ca. 50°C) in einem 100 ml-Messkolben gelöst. Clotrimazol wird unter Rühren zur obigen Lösung gegeben, danach wird mit Milchsäureethylester auf 100 ml aufgefüllt. Es wird weitergerührt, bis eine homogene Lösung entstanden ist.

### Beispiel 2: Clotrimazol-Teebaumöl-Lösung 1 % + 10 %

| | |
|---|---|
| Milchsäureethylester | 20,0 ml |
| Harnstoff | 2,0 g |
| Clotrimazol | 1,0 g |
| Teebaumöl | 10,0 g |
| Milchsäureethylester | ad 100,0 ml |

Harnstoff wird in 20 ml Milchsäureethylester unter Rühren und Erwärmen (ca. 50°C) in einem 100 ml-Messkolben gelöst, Anschliessend werden Clotrimazol und Teebaumöl unter Rühren zur obigen Lösung gegeben und mit Milchsäureethylester auf 100 ml aufgefüllt. Es wird solange weitergerührt, bis eine homogene Lösung entstanden ist.

### Beispiel 3: Teebaumöl-Lösung 30 %

| | |
|---|---|
| Milchsäureethylester | 44,0 g |
| Teebaumöl | 30,0 g |
| Lavendelöl | 6,0 g |
| Propylenglykol | 20,0 g |

Alle Substanzen werden in ein Becherglas eingewogen und solange gerührt, bis eine homogene Lösung entstanden ist.

### Beispiel 4: Niemextrakt-Teebaumöl-Lösung 12 %

| | |
|---|---|
| Niemextrakt | 2,0 g |
| Propylenglycol | 20,0 g |
| Milchsäureethylester | 63,0 g |
| Teebaumöl | 10,0 g |
| Lavendelöl | 5,0 g |

Niemextrakt wird in Propylenglykol unter Rühren gelöst. Anschliessend werden die restlichen Substanzen beigefügt. Die Mischung wird solange weitergerührt, bis eine homogene Lösung entstanden ist.

### Beispiel 5: Prolin-Lösung 1,5 %

| | |
|---|---|
| L-Prolin | 1,5 g |
| Propylenglycol | 65,0 g |
| Milchsäureethylester | 33,5 g |

L-Prolin wird in Propylenglykol unter Rühren und Erwärmen gelöst. Änschliessend wird Milchsäureethylester beigefügt und solange weitergerührt, bis eine homogene Lösung entstanden ist.

### Beispiel 6

| | |
|---|---|
| L-Prolin | 2,0 g |
| Äpfelsäurediäethylester | 98,0 g |

L-Prolin wird unter Rühren in Äpfelsäurediethylester eigetragen und bis zur vollständigen Auflösung nachgerührt.

### Beispiel 7

In der folgenden Tabelle sind weitere erfindungsgemässe Zusammensetzungen angegeben, die unter Verwendung von jeweils 50,0 g eines erfindungsgemäss als Carrier zu verwendenden Hydroxycarbonsäure- C₁-C₄-alkylesters und jeweils 1,0 g Wirkstoff erhalten werden. Zur Herstellung der Zusammensetzungen wurde jeweils der Wirkstoff unter Rühren bei Raumtemperatur oder mässig erhöhter Temperatur (~ 30°C - 50°C) in den Hydroxcarbonsäureester eingetragen. Je nach Wirkstoff entsteht nach 1-5-stündigem Nachrühren eine klare, unmittelbar gebrauchsfertige Lösung.

| **Wirkstoff** | **Hydroxycarbonsäureester** |
|---|---|
| Clotrimazol | Milchsäureethylester |
| Isoconazol | Milchsäureethylester |
| Ketoconazol | Milchsäureethylester |
| Itraconazol | Milchsäureethylester |
| Clotrimazol | Zitronensäuretriethylester |
| Isoconazol | Zitronensäuretriethylester |
| Ketoconazol | Zitronensäuretriethylester |
| Itraconazol | Zitronensäuretriethylester |
| Clotrimazol | Äpfelsäurediisopropylester |
| Isoconazol | Äpfelsäureidisopropylester |
| Ketoconazol | Äpfelsäurediisopropylester |
| Itraconazol | Äpfelsäurediisopropylester |

### Beispiel 8

In der folgenden Tabelle sind weitere erfindungsgemässe Zusammensetzungen angegeben, die unter Verwendung von jeweils 88,0 g eines erfindungsgemäss zu als Carrier zu verwendenden Hydroxycarbonsäure-C₁-C₄-alkylesters und 12,0 g einer aus 5 g Lavendelöl, 5 g Teebaumöl und 2 g Niembaumblatt-Extrakt bestehenden Wirkstoffkombination erhalten werden. Die Zusammensetzungen wurden durch Einrühren der Wirkstoffkombination in den jeweils verwendeten Hydroxycarbonsäureester bei Raumtemperatur hergestellt. Die so erhaltene Zusammensetzung ist unmittelbar gebrauchsfertig.

| **Wirkstoff** | **Hydroxycarbonsäureester** |
|---|---|
| Lavendelöl/lTeebaumöIINiembaumblatt-Extrakt | Milchsäureethylester |
| Lavendelöl/Teebaumöl/Niembaumblatt-Extrakt | Zitronensäuretriethylester |
| Lavendelöl/Teebaumöl/Niembaumblatt-Extrakt | Äpfelsäurediisopropylester |

## Patentansprüche

1. Wasserfreie und von film- und lackbildenden Zusätzen freie, topisch anwendbare Mittel zur Behandlung von Onychomykosen und zur Nagelpflege, enthaltend
a) eine oder mehrere Wirksubstanzen,
b) einen oder mehrere C₁-C₄-Alkylester der Milchsäure, der Äpfelsäure, der Weinsäure oder der Zitronensäure als Carrier und
c) gegebenenfalls physiologisch verträgliche Hilfsstoffe.

2. Topisch anwendbares Mittel gemäss Anspruch 1, **dadurch gekennzeichnet, dass** es einen Ethylester der Milchsäure, der Äpfelsäure, der Weinsäure oder der Zitronensäure als Carrier enthält.

3. Topisch anwendbares Mittel gemäss Anspruch 1, **dadurch gekennzeichnet, dass** es einen Isopropylester der Milchsäure, der Äpfelsäure, der Weinsäure oder der Zitronensäure als Carrier enthält.

4. Topisch anwendbares Mittel gemäss einem der Ansprüche 1 oder 2, **dadurch gekennzeichnet, dass** es Milchsäureethylester als Carrier enthält.

5. Topisch anwendbares Mittel gemäss einem der Ansprüche 1 oder 3, **dadurch gekennzeichnet, dass** es Äpfelsäurediisopropylester als Carrier enthält.

6. Topisch anwendbares Mittel gemäss einem der Ansprüche 1 bis 5, **gekennzeichnet durch** einen Gehalt an einer oder mehrerer aus der aus Antimykotika synthetischer und natürlicher Herkunft, Antibiotika, Antiseptika, Kortikosteroiden und Nähr- und Aufbaustoffen bestehenden Gruppe ausgewählten Wirksubstanzen.

7. Topisch anwendbares Mittel gemäss einem der Ansprüche 1 bis 5, **gekennzeichnet durch** einen Gehalt an einer oder mehreren antimykotischen Wirksubstanzen, die aus (±)-cis-2,6-Dimethyl-4-[2-methyl-3-(*p*-*tert*-pentyl-phenyl)propyl]morpholin (Amorolfin), Amphotericin, 6-Cyclohexyl-1-hydroxy-4-methyl-2(1H)pyridinon (Ciclopirox), Bis-phenyl-(2-chlorphenyl)-1-imidazolylmethan (Clotrimazol), 1-[2-(2,4-Dichlorphenyl)-2-(4-chlorbenzyloxy)-ethyl]-imidazol (Econazol), 2,4-Difluor-α,α-bis(1*H*-1,2,4-triazol-1-ylmethyl)benzylalkohol (Fluconazol), 5-Fluorcytosin (Flucytosin), 7-Chlor-trimethoxy-methylspiro-[benzofuran-cyclohexen]-dion (Griseofulvin), 1-[2,4-Dichlor-β-(2,6-dichlorbenzyloxy)-phenethyl]-imidazol (Isoconazol), (±)-1-*sec*-Butyl-4-{4-[4-(4-{[(2R*,4S*)-2-{2,4-dichlorphenyl)-2-(1,2,4-triazol-1-ylmethyl)-1,3-dioxolan-4-yl]methoxy)-phenyl)-1-piperazinyl]phenyl}-4,5-dihydro-1,2,4-triazol-5-on (Itraconazol), (±)-cis-1-Acetyl-4-{4-([2-(2,4-dichlorphenyl)-2-(1H-imidazol-1-ylmethyl)-1,3-dioxolan-4-yl]methoxy)phenyl}piperazin (Ketoconazol), 1-[2,4-Dichlor-β-(2,4-dichlorbenzyloxyl)-phenethyl]-imidazol (Miconazol), (*E*)-N-Cinnamyl-*N*-methyl-1-naphthylmethylamin (Naftifin), Nystatin, (*E*)-*N*-(6,6-Dimethyl-2-hepten-4-inyl)-*N*-methyl-1-naphthylmethylamin (Terbinafin), 1[2-{(2-Chlor-3-thienyl)methoxy}-2-(2,4-dichlorphenyl)ethyl]-1H-imidazol (Tioconazol), O-2-Naphthyl-N-methyl-N-(3-tolyl)-thiocarbamat (Tolnaftat) und α-(2,4-Difluorophenyl)-5-fluoro-β-methyl-α-(1H-1,2,4-triazol-1-ylmethyl)-4-pyrimidinethanol (Voriconazol) ausgewählt sind.

8. Topisch anwendbares Mittel gemäss einem der Ansprüche 1 bis 5, **gekennzeichnet durch** einen Gehalt an einer oder mehrerer antibakterieller oder antimykotischer Wirksubstanzen, die aus Teebaumöl, Lavendelöl, Thujaöl und Blattextrakt des Niembaumes ausgewählt sind.

9. Topisch anwendbares Mittel gemäss einem der Ansprüche 1 bis 5, **gekennzeichnet durch** einen Gehalt an einer oder mehrerer antibiotischer Wirksubstanzen, die aus α-Amino-4-hydroxybenzylpenicillin (Amoxicillin), D-(-)-α-Aminobenzylpenicillin (Ampicillin), 3,3-Dimethyl-7-oxo-6-phenylacetamido-4-thia-1-azabicyclo-[3.2.0]-heptan-2-carbonsäure (Benzylpenicillin), Benzylpenicillin-Benzathin, 3-Chloro-7-D(2-phenylglycinamido)-cephalosporansäure (Cefaclor), 7β-[D-2-Amino-(4-hydroxyphenyl)-acetylamino]-3-methyl-cephalosporansäure (Cefadroxil), Amino-phenylacetamido-methyl-cephalosporansäure (Cefalexin), D(-)-threo-2-dichloracetamido-1-(4-nitrophenyl)-1,3-propandiol (Chloramphenicol), 1-Cyclopropyl-6-fluor-1,4-dihydro-4-oxo-7-(1-piperazinyl)-3-chinolincarbonsäure (Ciprofloxacin), (Z)-(2R,5R)-3-(2-Hydroxyethyliden)-7-oxo-4-oxa-1-azabicyclo[3.2.0]heptan-2-carbonsäure (Clavulansäure), 7-Chlor-7-desoxy-lincomycin (Clindamycin), 6-Desoxy-5-hydroxytetracyclin (Doxycyclin), 1-Ethyl-6-fluor-1,4-dihydro-4-oxo-7-(1-piperazinyl)-1,8-naphthyridin-3-carbonsäure (Enoxacin), Erythromycin, 3-(2-Chlor-6-fluorphenyl)-5-methyl-4-isoxazolyl-penicillin (Flucloxacillin), Kanamycin, Lincomycin, 7-Dimethylamino-6-desoxy-6-desmethyltetracyclin (Minocyclin), 6-(2-ethoxy-1-naphthamido)-penicillin (Nafcillin), 1-Ethyl-1,4-dihydro-7-methyl-4-oxo-1,8-naphthyridin-3-carbonsäure (Nalidixinsäure), Neomycin, 1-Ethyl-6-fluoro-1,4-dihydro-4-oxo-7-(1-piperazinyl)-3-chinolincarboxylsäure (Norfloxacin), (±)-9-Fluor-2,3-dihydro-3-methyl-10-(4-rnethyl-1-piperazinyl)-7-oxo-7*H*-pyrido[1,2,3-*de*][1,4]benzoxazin-6-carbonsäure (Ofloxacin), 6-(5-Methyl-3-phenyl-4-isoxazolcarboxamido)penicillansäure (Oxacillin), 6-Phenoxyacetylamino-penicillansäure (Phenoxymethylpenicillin) und 4-Dimethylamino-actahydro-pentahydroxy-1, 11-dioxo-6-methylnaphtacen-2-carbamid (Tetracyclin) ausgewählt sind.

10. Topisch anwendbares Mittel gemäss einem der Ansprüche 1 bis 5, **gekennzeichnet durch** einen Gehalt an einer oder mehrerer antiseptischer Wirksubstanzen, die aus Alkylbenzyldimethylammoniumchlorid (Benzalkoniumchlorid), N-Benzyl-N,N-dimethyl-2-{2-[p-(1,1,3,3,tetramethylbuthyl)-phenoxy]-ethoxy}-ethylammoniumhydroxid (Benzethoniumchlorid), Cetyltrimethylammoniumhydroxid (Cetrimoniumbromid), 1,1'-Hexamethylen-bis-[5-(p-chlorphenyl)-biguanid] (Chlorhexidin), N¹,N¹-Dekamethylen-bis-(4-aminochinaldiniumhydroxid) (Dequaliniumchlorid), N-(4-Chlorphenyl)-N'-(3,4-dichlorphenyl)harnstoff (Triclocarban) und 5-Chlor-2-(2,4-dichlorphenoxy)phenol (Triclosan) ausgewählt sind.

11. Topisch anwendbares Mittel gemäss einem der Ansprüche 1 bis 5, **gekennzeichnet durch** einen Wirkstoffgehalt an einem oder mehreren Kortikosteroiden, die aus 9α-Chlor-16β-methylprednisolon (Beclamethason), 9-Fluor-11β,17,21-trihydroxy-16β-methyl-1,4-pregnadien-3,20-dion (Betamethason), 21-Chlor-9-fluor-11β,17-dihydroxy-16β-methyl-1,4-pregnadien-3,20-dion (Clobetasol), 17,21-Dihydroxy-pregn-4-en-3,11,20-trion (Cortison), 11β,16α,17α,21-Tetrahydroxy-1,4-pregnadien-3,20-dion-16,17-acetonacetal (Desonid), 9-Fluor-11β-17,21-trihydroxy-16α-methylpregna-1,4-dien-3,20-dion (Dexamethason), 9α,11β-Dichloro-6α-fluoro-21-hydroxy-16α,17α-(isopropylidendioxy)-pregna-1,4-dien-3,20-dion (Flucloronid), 6α,9α-Difluor-16α,17α-isopropylidendioxy-corticosteron (Fluocinolonacetonid), 6α,9α-Difluor-16α, 17α-isopropylidendioxy-corticosteron-acetat (Fluocinonid), 6α-Fluor-11β,21-dihydroxy-16α, 17-isopropylidendioxy-4-pregnen-3,20-dion (Fludroxycortid), 3-(2-chloroethoxy)-9α-fluoro-6-formyl-11β,21-dihydroxy-16α,17α-isopropylidendioxypregna-3,5-dien-20-on (Formocortal), 21-Chlor-9α-fluor-11β-hydroxy-16α, 17α-isopropylidendioxy-4-pregnen-3,20-dion (Halcinonid), 17α-Hydroxycorticosteron (Hydrocortison), 11β,17,21-Trihydroxy-6α-methyl-1,4-pregnadien-3,20-dion (Methylprednisolon), 11β,17,21-Trihydroxy-pregna-1,4-dien-3,20-dion (Prednisolon), 17α,21-Dihydroxypregna-1,4-dien-3,11,20-trion (Prednison), 9-Fluor-16α-hydroxyprednisolon (Triamcinolon) und Triamcinolon-16α,17α-acetonid (Triamcinolonacetonid) ausgewählt sind.

12. Topisch anwendbares Mittel gemäss einem der Ansprüche 1 bis 5, **gekennzeichnet durch** einen Gehalt an dem Nähr- und Aufbaustoff L-Prolin.

13. Topisch anwendbares Mittel gemäss einem der Ansprüche 1 bis 5, **gekennzeichnet durch** einen Gehalt an L-Prolin in Kombination mit einem oder mehreren weiteren Nähr- und Aufbaustoffen, ausgewählt aus der Gruppe der Aminosäuren, den Vitaminen und den Mineralstoffen.

14. Topisch anwendbares Mittel gemäss einem der Ansprüche 1 bis 5, **gekennzeichnet durch** einen Gehalt an L-Prolin in Kombination mit einem oder mehreren Nähr- und Aufbaustoffen, ausgewählt aus Lysin, Cystein, Gelatine, Biotin, Panthenol, Dexpanthenol und anorganischen oder organischen Calcium-, Magnesium- oder Zinkverbindungen.

15. Topisch anwendbares Mittel gemäss einem der Ansprüche 1 bis 5, **gekennzeichnet durch** einen Gehalt an einem oder mehreren Hilfsstoffen, die aus der aus Terpenen oder Terpene enthaltenden Ölen, Alkoholen, Ketonen, Fettsäureestern, Polyglykolen, Tensiden, Harnstoff, Antioxidantien und Komplexierungsmitteln bestehenden Gruppe ausgewählt sind.

16. Topisch anwendbares Mittel gemäss einem der Ansprüche 1-15, **dadurch gekennzeichnet, dass** 0,01 bis 20 Gewichtsprozent an einer oder mehrerer Wirksubstanzen, 1 bis 99,99 Gewichtsprozent an C₁-C₄-Alkylester der Milchsäure, der Äpfelsäure, der Weinsäure oder der Zitronensäure und 0 bis 98,99 Gewichtsprozent Hilfsstoffe enthalten sind.

17. Verfahren zur Herstellung von topisch anwendbaren Mitteln gemäss einem der Ansprüche 1-16, **dadurch gekennzeichnet, dass** man einen oder mehrere C₁-C₄-Alkylester der Milchsäure, der Äpfelsäure, der Weinsäure oder der Zitronensäure und gewünschtenfalls einen oder mehrere Hilfsstoffe homogen vermischt, danach eine oder mehrere Wirksubstanzen in der Mischung unter Rühren und gegebenenfalls Erwärmen löst und solange weiterrührt bis eine homogene Lösung entstanden ist.

18. Verfahren zur Herstellung eines topisch anwendbaren Mittels gemäss einem der Ansprüche 1-16, **dadurch gekennzeichnet, dass** man die Lösung nach Anspruch 16 unter Zugabe von physiologisch verträglichen Formulierungshilfsstoffen nach üblichen Verfahren zu topischen Darreichungsformen weiterverarbeitet.

19. Verwendung eines topisch anwendbaren Mittels gemäss einem der Ansprüche 1-11 und 15-16 zur Behandlung, Prävention, Nachbehandlung oder unterstützenden Behandlung von Nagelerkrankungen und periungualen Erkrankungen.

20. Verwendung eines topisch anwendbaren Mittels gemäss einem der Ansprüche 1-6 und 12-16 zur Nagelpflege.

21. Verwendung eines topisch anwendbaren Mittels gemäss einem der Ansprüche 1-11 und 15-16 zur Behandlung von Pilzinfektionen der Hufe, Klauen und Krallen von Haus- und Nutztieren.

## Claims

1. Topical application products free of water and film- and lacquer-forming additives for the treatment of onychomycoses and nail care containing
a) one or more active substances
b) one or more C₁-C₄-alkyl esters of lactic acid, malic acid, tartaric acid or citric acid as carriers and
c) optionally physiologically compatible adjuvants.

2. Topical application product according to claim 1, **characterized in that** it contains an ethyl ester of lactic acid, malic acid, tartaric acid or citric acid as carrier.

3. Topical application product according to claim 1, **characterized in that** it contains an isopropyl ester of lactic acid, malic acid, tartaric acid or citric acid as carrier.

4. Topical application product according to one of claims 1 or 2, **characterized in that** it contains lactic acid ethyl ester as carrier.

5. Topical application product according to one of claims 1 or 3, **characterized in that** it contains malic acid diisopropylester as carrier.

6. Topical application product according to one of claims 1 to 5, **characterized by** a content of one or more active substances selected from the group consisting of antimycotics of synthetic or natural origin, antibiotics, antiseptics, corticosteroids and nutrient and anabolic substances.

7. Topical application product according to one of claims 1 to 5, **characterized by** a content of one or more antimycotic active substances selected from the group consisting of (±)-cis-2,6-dimethyl-4-[2-methyl-3-(*p*-*tert*-pentylphenyl)propyl]morpholine (amorolfin), amphotericin, 6-cyclohexyl-1-hydroxy-4-methyl-2(1H)pyridinone (ciclopirox), bis-phenyl-(2-chlorophenyl)-1-imidazolylmethane (clotrimazol), 1-[2-(2,4-dichlorophenyl)-2-(4-chlorobenzyloxy)-ethyl]-imidazole (econazol), 2,4-difluoro-α,α-bis(1H-1,2,4-triazol-1-ylmethyl)benzylalcohol (fluconazol), 5-fluorocytosine (flucytosin), 7-chlorotrimethoxy-methylspiro-[benzofuran-cyclohexen]-dione (griseofulvin), 1-[2,4-dichloro-β-(2,6-dichlorobenzyloxy)-phenethyl]-imidazole (isoconazol), (±)-1-*sec*-butyl-4-{4-[4-(4-{[(2R*,4S*)-2-(2,4-dichlorophenyl)-2-(1,2,4-triazol-1-ylmethyl)-1,3-dioxolan-4-yl]methoxy}-phenyl)-1-piperazinyl]phenyl}-4,5-dihydro-1,2,4-triazol-5-one (itraconazol), (±)-cis-1-acetyl-4-{4-([2-(2,4-dichlorophenyl)-2-(1H-imidazol-1-ylmethyl)-1,3-dioxolan-4-yl]methoxy)phenyl}piperazine (ketoconazol), 1-[2,4-dichloro-β-(2,4-dichlorobenzyloxyl)-phenethyl)-imidazole(miconazol), (*E*)-N-cinnamyl-*N*-methyl-1-naphthylmethylamine (naftifin), nystatin, (*E*)-N-(6,6-dimethyl-2-hepten-4-ynyl)-*N*-methyl-1-naphthylmethylamine (terbinafin), 1[2-{(2-chloro-3-thienyl)methoxy}-2-(2,4-dichlorophenyl)ethyl]-1 H-imidazole (tioconazol), O-2-naphthyl-N-methyl-N-(3-tolyl)-thiocarbamate (tolnaftat) and α-(2,4-difluorophenyl)-5-fluoro-β-methyl-α-(1H-1,2,4-triazol-1-ylmethyl)-4-pyrimidinethanol (voriconazol).

8. Topical application product according to one of claims 1 to 5, **characterized by** a content of one or more antibacterially or antimycotically active substances selected from tea tree oil, lavender oil, thuja oil and leaf extract of the nim tree.

9. Topical application product according to one of claims 1 to 5, **characterized by** a content of one or more antibiotic substances selected from α-amino-4-hydroxybenzylpenicillin (amoxicillin), D-(-)-α-aminobenzylpenicillin (ampicillin), 3,3-dimethyl-7-oxo-6-phenylacetamido-4-thia-1-azabicyclo-[3.2.0]-heptan-2-carboxylic acid (benzylpenicillin), benzylpenicillin-benzathin, 3-chloro-7-D-(2-phenylglycinamido)-cephalosporanic acid (cefaclor), 7β-[D-2-amino-(4-hydroxyphenyl)-acetylamino]-3-methyl-cephalosporanic acid (cefadroxil), aminophenylacetamido-methyl-cephalosporanic acid (cefalexin), D(-)-threo-2-dichloroacetamido-1-(4-nitrophenyl)-1,3-propanediol (chloramphenicol), 1-cyclopropyl-6-fluoro-1,4-dihydro-4-oxo-7-(piperazinyl)-3-quinolinecarboxylic acid (ciprofloxacin), (Z)-(2R,5R)-3-(2-hydroxyethylidene)-7-oxo-4-oxa-1-azabicyclo[3.2.0]heptan-2-carboxylic acid (clavulanic acid), 7-chloro-7-desoxylincomycin (clindamycin), 6-desoxy-5-hydroxytetracycline (doxycyclin), 1-ethyl-6-fluoro-1,4-dihydro-4-oxo-7-(1-piperazinyl)-1,8-naphthyridin-3-carboxylic acid (enoxacin), erythromycin, 3-(2-chloro-6-fluorophenyl)-5-methyl-4-isoxazolylpenicillin (flucloxacillin), kanamycin, lincomycin, 7-dimethylamino-6-desoxy-6-desmethyltetracycline (minocycline), 6-(2-ethoxy-1-naphthamido)-penicillin (nafcillin), 1-ethyl-1,4-dihydro-7-methyl-4-oxo-1,8-naphthyridin-3-carboxylic acid (nalidixic acid), neomycin, 1-ethyl-6-fluoro-1,4-dihydro-4-oxo-7-(1-piperazinyl)-3-quinolinecarboxylic acid (norfloxacin), (±)-9-fluoro-2,3-dihydro-3-methyl-10-(4-methyl-1-piperazinyl)-7-oxo-7*H*-pyrido[1,2,3-*de*][1,4]benzoxazin-6-carboxylic acid (ofloxacin), 6-(5-methyl-3-phenyl-4-isoxazolcarboxamido)penicillanic acid (oxacillin), 6-phenoxyacetylamino-penicillanic acid (phenoxymethylpenicillin) and 4-dimethylamino-octahydro-pentahydroxy-1,11-dioxo-6-methyl-naphtacene-2-carbamide (tetracyclin).

10. Topical application product according to one of claims 1 to 5, **characterized by** a content of one or more antiseptically active substances selected from alkylbenzyldimethylammonium chloride (benzalkonium chloride), N-benzyl-N,N-dimethyl-2-{2-[p-(1,1,3,3,-tetramethylbutyl)-phenoxy]-ethoxy}-ethylammonium hydroxide (benzethonium chloride), cetyltrimethylammonium hydroxide (cetrimonium bromide), 1,1'-hexamethylen-bis-[5-(p-chlorophenyl)-biguanide] (chlorohexidine), N¹, N¹-decamethylen-bis-(4-aminoquinaldinium hydroxide) (dequalinium chloride), N-(4-chlorophenyl)-N'-(3,4-dichlorophenyl)urea (triclocarbane) and 5-chloro-2-(2,4-dichlorophenoxy)phenol (triclosan).

11. Topical application product according to one of claims 1 to 5, **characterized by** an active ingredient content of one or more corticosteroids selected from 9α-chloro-16β-methylprednisolone (beclomethasone), 9-fluoro-11β, 17, 21-trihydroxy-16β-methyl-1,4-pregnadien-3,20-dione (betamethason), 21-chloro-9-fluoro-11β, 17-dihydroxy-16β-methyl-1,4-pregnadien-3,20-dione (clobetasol), 17,21-dihydroxy-pregn-4-en-3,11,20-trione (cortisone), 11β,16α,17α,21-tetrahydroxy-1,4-pregnadien-3,20-dione-16,17-acetone acetal (desonid), 9-fluoro-11β-17,21-trihydroxy-16α-methylpregna-1,4-dien-3,20-dione (dexamethason), 9α,11β-dichloro-6α-fluoro-21-hydroxy-16α,17α-(isopropylidenedioxy)-pregna-1,4-dien-3,20-dione (flucloronid), 6α,9α-difluoro-16α,17α-isopropylidenedioxy-corticosterone (fluocinolonacetonide), 6α, 9α-difluoro-16α, 17α-isopropylidenedioxy-corticosterone-acetate (fluocinonid), 6α-ftuoro-11β, 21-dihydroxy-16α,17-isopropylidenedioxy-4-pregnen-3,20-dione (fludroxycortide), 3-(2-chloroethoxy)-9α-fluoro-6-formyl-11β,21-dihydroxy-16α,17α-isopropylidenedioxypregna-3,5-dien-20-one (formocortal), 21-chloro-9α-fluoro-11β-hydroxy-16α,17α-isopropylidenedioxy-4-pregnen-3,20-dione (halcinonide), 17α-hydroxycorticosterone (hydrocortisone), 11β, 17,21-trihydroxy-6α-methyl-1,4-pregnadien-3,20-dione (methylprednisolone), 11β, 17,21-trihydroxy-pregna-1,4-dien-3,20-dione (prednisolone), 17α, 21-dihydroxypregna-1,4-dien-3,11,20-trione (prednisone), 9-fluoro-16α-hydroxyprednisolone (triamcinolone) and triamcinolone-16α,17-acetonide (triamcinolone acetonide).

12. Topical application product according to one of claims 1 to 5, **characterized by** a content of the nutrient and anabolic substance L-proline.

13. Topical application product according to one of claims 1 to 5, **characterized by** a content of L-proline in combination with one or more further nutrient and anabolic substances selected from the group of the amino acids, the vitamins and the mineral substances.

14. Topical application product according to one of claims 1 to 5, **characterized by** a content of L-proline in combination with one or more nutrient and anabolic substances selected from lysine, cystein, gelatine, biotin, panthenol, dexpanthenol and inorganic or organic calcium, magnesium or zinc compounds.

15. Topical application product according to one of claims 1 to 5, **characterized by** a content of one or more adjuvants from the group consisting of terpenes or terpene-containing oils, alcohols, ketones, fatty acid esters, polyglycols, tensides, urea, antioxidants and complexing agents.

16. Topical application product according to one of claims 1 to 15, **characterized in that** it contains 0.01 to 20 percent by weight of one or more active substances, 1 to 99.99 percent by weight of C₁-C₄-alkyl esters of lactic acid, malic acid, tartaric acid or citric acid and 0 to 98.99 percent by weight of the adjuvants.

17. Process for the manufacture of topical application products according to one of claims 1 to 16, **characterized in that** one or more C₁-C₄-alkyl esters of lactic acid, malic acid, tartaric acid or citric acid and, if desired, one or more adjuvants are homogenously mixed, subsequently one or more active substances are dissolved in the mixture with stirring and optional heating, and stirring is continued until a homogenous solution is obtained.

18. Process for the manufacture of a topical application product according to one of claims 1 to 16, **characterized in that** the solution according to claim 16 is further processed with the addition of physiologically acceptable formulation adjuvants to topical application forms.

19. Use of a topical application product according to one of claims 1-11 and 15 -16 for the treatment, prevention, after-treatment and supporting treatment of nail diseases and periungual diseases.

20. Use of a topical application product according to one of claims 1-6 and 12-16 for nail care.

21. Use of a topical application product according to one of claims 1-11 and 15-16, for the treatment of mycotic infections of the hooves, paws and claws of pets and domestic animals

## Revendications

1. Produits d'application topique, anhydres, exempts d'additifs formant une pellicule ou un vernis, pour le traitement des onychomycoses et pour les soins des ongles, contenant
a) une ou plusieurs substances actives,
b) un ou plusieurs esters d'alkyle C₁-C₄ de l'acide lactique, de l'acide malique, de l'acide tartrique ou de l'acide citrique en tant que carrier (substance porteuse) et
c) le cas échéant, des substances auxiliaires physiologiquement bien tolérées.

2. Produit d'application topique selon la revendication 1, **caractérisé en ce qu'**il contient un ester éthylique de l'acide lactique, de l'acide malique, de l'acide tartrique ou de l'acide citrique en tant que substance porteuse.

3. Produit d'application topique selon la revendication 1, **caractérisé en ce qu'**il contient un ester isopropylique de l'acide lactique, de l'acide malique, de l'acide tartrique ou de l'acide citrique en tant que substance porteuse.

4. Produit d'application topique selon la revendication 1 ou 2, **caractérisé en ce qu'**il contient de l'ester éthylique de l'acide lactique en tant que substance porteuse.

5. Produit d'application topique selon la revendication 1 ou 3, **caractérisé en ce qu'**il contient de l'ester diisopropylique de l'acide malique en tant que substance porteuse.

6. Produit d'application topique selon l'une des revendications 1 à 5, **caractérisé par** sa teneur en une ou plusieurs substances actives issues d'un groupe composé d'antimycosiques d'origine synthétique ou naturelle, d'antibiotiques, d'antiseptiques, de corticostéroïdes, ainsi que de substances nutritives et reconstituantes.

7. Produit d'application topique selon l'une des revendications 1 à 5, **caractérisé par** sa teneur en une ou plusieurs substances actives antimycosiques choisies parmi les suivantes: (±)-cis-2,6-dimethyl-4-[2-methyl-3-(*p*-*tert*-pentylphenyl)propyl]morpholine (amorolfin), amphotericin, 6-cyclohexyl-1-hydroxy-4-methyl-2(1H)pyridinone (ciclopirox), bis-phenyl-(2-chlorophenyl)-1-imidazolylmethane (clotrimazol), 1-[2-(2,4-dichlorophenyl)-2-(4-chlorobenzyloxy)-ethyl]-imidazole (econazol), 2,4-difluoro-α,α-bis(1H-1,2,4-triazol-1-ylmethyl)benzylalcohol (fluconazol), 5-fluorocytosine (flucytosin), 7-chlorotrimethoxy-methylspiro-[benzofuran-cyclohexen]-dione (griseofulvin), 1-[2,4-dichloro-β-(2,6-dichlorobenzyloxy)-phenethyl]-imidazole (isoconazol), (±)-1-*sec*-butyl-4-{4-[4-(4-{[(2R*,4S*)-2-(2,4-dichlorophenyl)-2-(1,2,4-triazol-1-ylmethyl)-1,3-dioxolan-4-yl]methoxy}-phenyl)-1-piperazinyl]phenyl}-4,5-dihydro-1,2,4-triazol-5-one (itraconazol), (±)-cis-1-acetyl-4-{4-([2-(2,4-dichlorophenyl)-2-(1H-imidazol-1-ylmethyl)-1,3-dioxolan-4-yl]methoxy)phenyl}piperazine (ketoconazol), 1-[2,4-dichloro-β-(2,4-dichlorobenzyloxyl)-phenethyl]-imidazole(miconazol), (*E*)-N-cinnamyl-*N*-methyl-1-naphthylmethylamine (naftifin), nystatin, (*E*)-N-(6,6-dimethyl-2-hepten-4-ynyl)-*N*-methyl-1-naphthylmethylamine (terbinafin), 1[2-{(2-chloro-3-thienyl)methoxy}-2-(2,4-dichlorophenyl)ethyl]-1 H-imidazole (tioconazol), O-2-naphthyl-N-methyl-N-(3-tolyl)-thiocarbamate (tolnaftat) and α-(2,4-difluorophenyl)-5-fluoro-β-methyl-α-(1H-1,2,4-triazol-1-ylmethyl)-4-pyrimidinethanol (voriconazol).

8. Produit d'application topique selon l'une des revendications 1 à 5, **caractérisé par** sa teneur en une ou plusieurs substances actives antibactériennes ou antimycosiques, choisies parmi l'huile essentielle de Melaleuca alternifolia, l'huile essentielle de lavande, l'huile essentielle de thuya et l'extrait de feuilles de margousier.

9. Produit d'application topique selon l'une des revendications 1 à 5, **caractérisé par** sa teneur en une ou plusieurs substances actives antibiotiques choisies parmi les suivantes: α-amino-4-hydroxybenzylpenicillin (amoxicillin), D-(-)-α-aminobenzylpenicillin (ampicillin), 3,3-dimethyl-7-oxo-6-phenylacetamido-4-thia-1-azabicyclo-[3.2.0]-heptan-2-carboxylic acid (benzylpenicillin), benzylpenicillin-benzathin, 3-chloro-7-D-(2-phenylglycinamido)-cephalosporanic acid (cefaclor), 7β-[D-2-amino-(4-hydroxyphenyl)-acetylamino]-3-methyl-cephalosporanic acid (cefadroxil), amino-phenylacetamido-methyl-cephalosporanic acid (cefalexin), D(-)-threo-2-dichloroacetamido-1-(4-nitrophenyl)-1,3-propanediol (chloramphenicol), 1-cyclopropyl-6-fluoro-1,4-dihydro-4-oxo-7-(piperazinyl)-3-quinolinecarboxylic acid (ciprofloxacin), (Z)-(2R,5R)-3-(2-hydroxyethylidene)-7-oxo-4-oxa-1-azabicyclo[3.2.0]heptan-2-carboxylic acid (clavulanic acid), 7-chloro-7-desoxylincomycin (clindamycin), 6-desoxy-5-hydroxytetracycline (doxycyclin), 1-ethyl-6-fluoro-1,4-dihydro-4-oxo-7-(1-piperazinyl)-1,8-naphthyridin-3-carboxylic acid (enoxacin), erythromycin, 3-(2-chloro-6-fluorophenyl)-5-methyl-4-isoxazolylpenicillin (flucloxacillin), kanamycin, lincomycin, 7-dimethylamino-6-desoxy-6-desmethyltetracycline (minocycline), 6-(2-ethoxy-1-naphthamido)-penicillin (nafcillin), 1-ethyl-1,4-dihydro-7-methyl-4-oxo-1,8-naphthyridin-3-carboxylic acid (nalidixic acid), neomycin, 1-ethyl-6-fluoro-1,4-dihydro-4-oxo-7-(1-piperazinyl)-3-quinolinecarboxylic acid (norfloxacin), (±)-9-fluoro-2,3-dihydro-3-methyl-10-(4-methyl-1-piperazinyl)-7-oxo-7*H*-pyrido[1,2,3-*de*][1,4]benzoxazin-6-carboxylic acid (ofloxacin), 6-(5-methyl-3-phenyl-4-isoxazolcarboxamido)penicillanic acid (oxacillin), 6-phenoxyacetylamino-penicillanic acid (phenoxymethylpenicillin) and 4-dimethylamino-octahydro-pentahydroxy-1, 11 -dioxo-6-methyl-naphtacene-2-carbamide (tetracyclin).

10. Produit d'application topique selon l'une des revendications 1 à 5, **caractérisé par** sa teneur en une ou plusieurs substances actives antiseptiques choisies parmi les suivantes : alkylbenzyldimethylammonium chloride (benzalkonium chloride), N-benzyl-N,N-dimethyl-2-{2-[p-(1,1,3,3,-tetramethylbutyl)-phenoxy]-ethoxy}-ethylammonium hydroxide (benzethonium chloride), cetyltrimethylammonium hydroxide (cetrimonium bromide), 1,1'-hexamethylen-bis-[5-(p-chlorophenyl)-biguanide] (chlorohexidine), N¹, N¹-decamethylen-bis-(4-aminoquinaldinium hydroxide) (dequalinium chloride), N-(4-chlorophenyl)-N'-(3,4-dichlorophenyl)urea (triclocarbane) and 5-chloro-2-(2,4-dichlorophenoxy)phenol (triclosan).

11. Produit d'application topique selon l'une des revendications 1 à 5, **caractérisé par** sa teneur en substances actives d'un ou plusieurs corticostéroïdes choisis parmi les suivants : 9α-chloro- 16β-methylprednisolone (beclomethasone), 9-fluoro-11β, 17,21-trihydroxy-16β-methyl-1,4-pregnadien-3,20-dione (betamethason), 21-chloro-9-fluoro-11β, 17-dihydroxy-16β-methyl-1,4-pregnadien-3,20-dione (clobetasol), 17,21-dihydroxy-pregn-4-en-3,11,20-trione (cortisone), 11β, 16α, 17α,21-tetrahydroxy-1,4-pregnadien-3,20-dione-16,17-acetone acetal (desonid), 9-fluoro-11β-17,21-trihydroxy-16α-methylpregna-1,4-dien-3,20-dione (dexamethason), 9α,11β-dichloro-6α-fluoro-21-hydroxy-16α,17α-(isopropylidenedioxy)-pregna-1,4-dien-3,20-dione (flucloronid), 6α,9α-difluoro-16α,17α-isopropylidenedioxy-corticosterone (fluocinolonacetonide), 6α, 9α-difluoro-16α, 17α-isopropylidenedioxy-corticosterone-acetate (fluocinonid), 6α-fluoro-11β, 21-dihydroxy-16α,17-isopropylidenedioxy-4-pregnen-3,20-dione (fludroxycortide), 3-(2-chloroethoxy)-9α-fluoro-6-formyl-11β,21-dihydroxy-16α,17α-isopropylidenedioxypregna-3,5-dien-20-one (formocortal), 21-chloro-9α-fluoro-11β-hydroxy-16α,17α-isopropylidenedioxy-4-pregnen-3,20-dione (halcinonide), 17α-hydroxycorticosterone (hydrocortisone), 11β, 17,21-trihydroxy-6α-methyl-1,4-pregnadien-3,20-dione (methylprednisolone), 11β, 17,21-trihydroxy-pregna-1,4-dien-3,20-dione (prednisolone), 17α, 21-dihydroxypregna-1,4-dien-3,11,20-trione (prednisone), 9-fluoro-16α-hydroxyprednisolone (triamcinolone) and triamcinolone-16α,17-acetonide (triamcinolone acetonide).

12. Produit d'application topique selon l'une des revendications 1 à 5, **caractérisé par** sa teneur en L-proline, substance nutritive et reconstituante.

13. Produit d'application topique selon l'une des revendications 1 à 5, **caractérisé par** sa teneur en L-proline en combinaison avec une ou plusieurs autres substances nutritives et reconstituantes, choisies parmi le groupe des acides aminés, les vitamines et les sels minéraux.

14. Produit d'application topique selon l'une des revendications 1 à 5, **caractérisé par** sa teneur en L-proline en combinaison avec une ou plusieurs substances nutritives et reconstituantes, choisies parmi la lysine, la cystéine, la gélatine, la biotine, le panthénol, le dexpanthénol et des composés organiques ou anorganiques de calcium, de magnésium ou de zinc.

15. Produit d'application topique selon l'une des revendications 1 à 5, **caractérisé par** sa teneur en une ou plusieurs substances auxiliaires choisies parmi le groupe composé de terpènes ou d'huiles contenant des terpènes, d'alcools, de cétones, d'esters d'acide gras, de polyglycols, d'agents tensioactifs, d'urée, d'antioxydants et d'agents complexants.

16. Produit d'application topique selon l'une des revendications 1-15, **caractérisé en ce qu'**il contient un pourcentage en poids de 0,01 à 20 d'une ou plusieurs substances actives, un pourcentage en poids de 1 à 99,99 d'ester d'alkyle C₁-C₄ de l'acide lactique, de l'acide malique, de l'acide tartrique ou de l'acide citrique et un pourcentage en poids de 0 à 98,99 de substances auxiliaires.

17. Procédé pour la fabrication de produits d'application topique selon l'une des revendications 1-16, **caractérisé en ce que** l'on réalise un mélange homogène avec un ou plusieurs esters d'alkyle C₁-C₄ de l'acide lactique, de l'acide malique, de l'acide tartrique ou de l'acide citrique et, au choix, avec une ou plusieurs substances auxiliaires, que l'on dissout ensuite une ou plusieurs substances actives dans le mélange en remuant et, le cas échéant, en le chauffant, et que l'on continue à remuer jusqu'à ce que l'on obtienne une solution homogène.

18. Procédé pour la fabrication d'un produit d'application topique selon l'une des revendications 1-16, **caractérisé en ce que** l'on transforme en formes galéniques topiques la solution obtenue suivant la revendication 16, selon les procédés usuels, en y ajoutant des substances auxiliaires de formulation bien tolérées physiologiquement.

19. Emploi d'un produit d'application topique selon l'une des revendications 1-11 et 15-16, pour le traitement, la prévention, le suivi médical ou le traitement d'appoint des maladies de l'ongle et des affections péri-unguéales.

20. Emploi d'un produit d'application topique selon l'une des revendications 1-6 et 12-16, pour les soins des ongles.

21. Emploi d'un produit d'application topique selon l'une des revendications 1-11 et 15-16, pour le traitement des mycoses touchant les sabots, les griffes et les ongles des animaux domestiques et d'utilité.
